## Europäisches Patentamt

## European Patent Office

## Office européen des brevets

(19)

(11) Numéro de publication: **0 213 005**
**B1**

(12)

# FASCICULE DE BREVET EUROPÉEN

(45) Date de publication du fascicule du brevet:
**31.05.89**

(51) Int. Cl.⁴: **C 12 M 1/00, C 12 P 7/06**

(21) Numéro de dépôt: **86401496.0**

(22) Date de dépôt: **04.07.86**

(54) **Procédé de production d'anhydride carbonique et d'éthanol par fermentation continue, et appareillage de mise en oeuvre.**

(30) Priorité: **15.07.85 FR 8510793**

(43) Date de publication de la demande:
**04.03.87 Bulletin 87/10**

(45) Mention de la délivrance du brevet:
**31.05.89 Bulletin 89/22**

(84) Etats contractants désignés:
**AT BE CH DE FR GB IT LI LU NL SE**

(56) Documents cité:
**EP-A-0 015 690**
**FR-A-1 307 047**
**FR-A-2 495 637**
**GB-A-2 017 752**
**GB-A-2 113 711**
**US-A-4 413 058**

(73) Titulaire: **L'AIR LIQUIDE, SOCIETE ANONYME POUR L'ETUDE ET L'EXPLOITATION DES PROCEDES GEORGES CLAUDE, 75, Quai d'Orsay, F-75321 Paris Cédex 07 (FR)**

(72) Inventeur: **Ghommidh, Charles, 621, Avenue Paul Parguel, F-34100 - Montpellier (FR)**
Inventeur: **Galambre, Hélène, 7, rue Rondelet, F-34100 - Montpellier (FR)**
Inventeur: **Graindorge, Philippe, 117, Cours Gambetta, F-69003 - Lyon (FR)**
Inventeur: **Navarro, Jean- Marie, 2429, Avenue du Père Soulas Villa 133, F-34100 - Montpellier (FR)**
Inventeur: **Amen, Jean, 108, Bd de la Reine, F-78000 - Versailles (FR)**
Inventeur: **Cutayar, Jacques- Marcel, 4, rue Pierre Vaudenay Les Metz, F-78350 - Jouy- en- Josas (FR)**

(74) Mandataire: **Jacobson, Claude, L'AIR LIQUIDE, SOCIETE ANONYME POUR L'ETUDE ET L'EXPLOITATION DES PROCEDES GEORGES CLAUDE 75, quai d'Orsay, F-75321 Paris Cédex 07 (FR)**

## Description

La présente invention concerne un procédé de production d'anhydride carbonique et d'éthanol par fermentation continue et un type d'appareillage de mise en oeuvre.

Les prévisions annoncées sur la disponibilité des ressources énergétiques naturelles et les conséquences qui en découleraient ont favorisé une recrudescence d'intérêt porté à la production de composés énergétiques par voie fermentaire, d'éthanol en particulier.

Si, actuellement on ne peut plus mettre en doute la faisabilité technique de la production d'éthanol par fermentation continue, économiquement un certain nombre de contraintes pèsent sur le développement de cette technique. Ainsi le substrat: sucre, matières amylacées, doit être converti, de façon aussi complète que possible, en alcool avec un rendement approchant la production maximale théorique de 0,51 kg d'éthanol par kg de glucose; de même, la teneur finale en éthanol du jus de fermentation doit être supérieure à 50 g par litre, afin de mininiser le coût énergétique de la distillation et le coût de traitement des vinasses de distillerie.

Dans le domaine fermentaire deux types de microorganismes présentent un intérêt industriel: les levures, principalement du genre Saccharomyces, sont bien connues et utilisées depuis fort longtemps en brasserie, vinification... En outre, les bactéries appartenant au genre Zymomonas, suscitent, en raison de leur activité métabolique supérieure, un intérêt croissant.

La productivité et le rendement constituent les deux principaux paramètres qui permettent de juger de l'efficacité d'un procédé de fermentation. La vitesse de production étant étroitement liée à la quantité de microorganismes actifs présente dans les fermenteurs, les procédés de fermentation continus doivent pour accroître leur productivité augmenter la population microbienne active. Et, pour lutter contre les phénomènes de dilution, qui auraient tendance à diminuer la concentration microbienne, engendrés par des taux de dilution importants, ces procédés font appel à des techniques de rétention de microorganismes performants.

Parmi ces techniques, on peut citer des moyens naturels tels la floculation naturelle des microorganismes qui conduit à l'obtention d'agrégats microbiens de densité élevée. Les moyens artificiels sont nombreux tels l'immobilisation des microorganismes sur un garnissage statique ou sur un support dynamique; la rétention par des procédés à membranes: microfiltration, ultrafiltration; la décantation associée à un recyclage; la centrifugation avec recyclage; et la floculation induite des microorganismes.

De tous les moyens précédemment décrits, seuls la floculation et les procédés à membranes permettent d'atteindre des concentrations microbiennes très élevées. Appliqués à la fermentation alcoolique, ils accroissent les concentrations en levure jusqu'à 100 à 120 g/l de matière sèche. A ces concentrations, les levures occupent environ 50 % du volume de fermentation, au-delà apparaissent des problèmes de transfert de matière qui diminuent les performances de la fermentation. Les levures ayant généralement une vitesse spécifique de production d'éthanol de l'ordre de 0,5 g d'éthanol/g levure séche, il n'est guère possible d'enregistrer des productivités supérieures à 50 g éthanol/litre de fermenteur/heure.

L'idée de travailler à de telles densités microbiennes, mais avec des microorganismes plus performants, a conduit de nombreux chercheurs à mettre en oeuvre une bactérie anaérobie capable de fermenter le glucose en éthanol. Cette bactérie, Zymomonas mobilis, appartient à la famille des pseudomonadacées et présente une vitesse spécifique de production d'éthanol de 4 g d'éthanol/g biomasse séche par heure. Des essais de fermentation alcoolique à haute densité cellulaire utilisant Zymomonas mobilis ont montré qu'il était possible d'atteindre des concentrations cellulaires de 60 à 80 g/l.

Le procédé de fermentation alcoolique continu, utilisant Zymomonas, décrit dans le brevet américain US-4 443 544 met en oeuvre une concentration cellulaire par microfiltration tangentielle. Il a permis d'atteindre des performances intéressantes: concentration microbienne de 50 à 60 g/l, concentration de la solution alcoolique de 65 g/l, productivité en anhydride carbonique de 200 g/l.h et rendement de conversion de 0,46 g d'éthanol/g de glucose. Toutefois, ce procédé présente un certain nombre d'inconvénients, tels une mise en oeuvre délicate de la microfiltration tangentielle posant des difficultés au stade industriel, des problèmes de colmatage rencontrés au niveau du module de filtration, ainsi que la nécessité d'éviter toute contamination à cause de la non sélectivité du système de rétention microbienne. C'est pourquoi, on a recherché d'autres procédés de fermentation utilisant des moyens de rétention de microorganismes plus simples.

Ainsi, C.D Scott et coll. ont proposé un procédé de fermentation alcoolique continu mettant en oeuvre une souche de Zymononas mobilis floculant naturellement associée à une rétention par décantation externe: un décanteur placé en sortie de fermenteur assure la récupération des flocs microbiens évacués dans l'effluent et leur recyclage dans le fermenteur au moyen d'une pompe de recirculation. Avec ce procédé, les résultats sont moins bons que dans le cas précédent, ainsi la concentration microbienne n'est que de 30 g/l, la concentration de la solution alcoolique de 54 g/l, la productivité en éthanol et $CO_2$ atteint 120 g/l.h avec un rendement de conversion de 0,5 g d'éthanol/g de glucose; ceci s'explique par le fait que ce dernier procédé retient moins de microorganismes actifs. La pompe de recirculation utilisée pour recycler les flocs microbiens a pour effet de briser ces flocs qui deviennent de plus en plus petits, décantent de moins en moins et finissent par être évacués du fermenteur.

Afin d'augmenter les chances de développement industriel de la production d'éthanol par voie biologique, il est apparu nécessaire de concevoir un système de fermentation à la fois plus performant et plus simple que ceux décrits précédemment.

On a recherché un procédé et un appareillage répondant à certains critères, tels la rétention d'importantes quantités de microorganismes permettant d'atteindre une concentration microbienne supérieure à 50 g/l; la production d'une solution alcoolique titrant au moins 50 g/l d'éthanol avec une productivité supérieure à 120 g/l, et pouvant même atteindre 250 g/l.h avec une concentration de 30 - 40 g/litre; l'insensibilité aux contaminants et la suppression de l'étape de stérilisation du milieu. En outre, vus les problèmes occasionnés par la mise en oeuvre d'une décantation externe, la suppression de tout moyen mécanique mobile devait être envisagée.

Le brevet FR-1 307 047 décrit un procédé et une installation pour la fabrication de liquides alcoolisés, et en particulier de la bière, par fermentation en présence de cellules enzymiques de la levure de bière floculante. Suivant ce procédé la concentration en sucre, le long du liquide de fermentation, est obtenue en réduisant ou empêchant totalement l'écoulement en retour du moût, de manière à ce que le dit écoulement soit pratiquement uniforme et qu'il n'y ait pas de gradient de vitesse. Et, pour éviter une perte de levure avec la bière sortant de la tour de fermentation, celle-ci est terminée par une zone de séparation à plus grande section transversale, qui permet à la levure de se déposer et de retourner directement dans la tour.

On propose un procédé de production d'anhydride carbonique et d'éthanol par fermentation continue d'un milieu de culture inoculé par des microorganismes du type bactéries appartenant au genre Zymomonas floculante avec rétention et recyclage des microorganismes, la rétention étant réalisée par décantation-recyclage interne obtenue par un ralentissement de la vitesse de circulation des fluides, avec réduction de la turbulence dans la partie supérieure de la zone de fermentation, par un brusque élargissement de la section de la dite zone, caractérisé en ce que le milieu de culture est inoculé par une souche hautement floculante de Zymomonas mobolis, déposée à l'Institut Pasteur le 21 janvier 1985 sous le numéro d'ordre attribué par l'autorité de dépôt international I-411, et en ce que les gaz de fermentation sont collectés au centre de la partie supérieure de la zone de fermentation où la turbulence du milieu de fermentation est réduite, avec collecte et évacuation de l'anhydride carbonique produit à différents niveaux de la zone de fermentation.

La productivité du système est telle que la production d'anhydride carbonique représente un à deux volumes de $CO_2$ par volume de fermenteur et par minute; en conséquence, l'évacuation de l'anhydride carbonique doit être la plus efficace possible, car les risques de formation de mousse qui dépendent de la nature du milieu fermenté sont à considérer. De plus, la turbulence engendrée par la production de gaz perturbe la décantation des agrégats microbiens et donc leur rétention au sein de la zone de fermentation.

L'évacuation de l'anhydride carbonique impose un système de séparation triphasique: gaz, liquide, solide adéquat. Pour faciliter cette évacuation, on propose à différents niveaux de la zone de fermentation la canalisation des bulles d'anhydride carbonique produit et leur coalescence, la collecte et l'évacuation de l'anhydride carbonique produit et le recyclage des agrégats microbiens ayant décanté dans une zone ou la turbulence est très faible. Un tel moyen réduit la turbulence au sein de la zone de fermentation; et cette technique d'évacuation du $CO_2$ est applicable aux systèmes monoétagé, bi ou multiétagé.

Au démarrage du processus de fermentation, une fraction des gaz de fermentation contenant l'anhydride carbonique récupéré peut être recyclé à la base de la zone de fermentation.

Le milieu de fermentation est maintenu à une température comprise entre 20 et 50°C, de préférence 25 à 40°C, le milieu d'alimentation étant préchauffé à la même température. Le pH du milieu de fermentation est compris entre 3,7 et 6,5, de préférence maintenu entre 4,3 et 5,2, par apport d'une solution d'un agent alcalin.

Le milieu d'alimentation contenant un carbohydrate fermentescible constitué par tout type de substrat simple d'origine naturelle ou synthétique comme le lactose hydrolysé, saccharose à partir des betteraves, canne à sucre, hydrolysat d'amidon provenant de maïs ou blé.

On introduit le milieu d'alimentation à divers niveaux de la zone de fermentation, en dessous de la zone où la turbulence du milieu de fermentation est réduite; le niveau du liquide dans la zone de fermentation étant maintenu constant.

La formation de mousses est contrôlée par apport régulé d'un agent tensio-actif.

Afin d'obtenir, pour des raisons économiques, une teneur résiduelle en sucre de l'effluent la plus basse possible, et pour diminuer le coût global de distillation ainsi que celui du traitement des eaux, une teneur en alcool de l'effluent la plus élevée possible, il est avantageux de mettre en oeuvre le procédé dans au moins deux étages de fermentation connectés en série du même type, géométriquement semblables, fonctionnant dans les mêmes conditions, tout en assurant un transfert non asservi des agrégats microbiens d'un étage à l'étage suivant; ce transfert étant périodique ou continu. Cette solution ne nécessite aucun dispositif d'asservissement à l'état du système ou de contrôle des quantités transférées.

D'autre part, les volumes gazeux, étant inversement proportionnels à la pression, on peut envisager, afin de limiter la turbulence, de conduire la fermentation sous pression. La pression maximale admissible dépend de la tolérance du microorganisme à la concentration en anhydride carbonique dissous dans le milieu; des pressions inférieures à 3 bars peuvent être mises en oeuvre sans difficulté majeure. La conduite de la fermentation sous légère pressurisation peut être associée à l'évacuation de l'anhydride carbonique à différents niveaux de la zone de fermentation.

Le procédé peut avantageusement être mis en oeuvre dans un fermenteur tubulaire, d'axe vertical, comportant en son sommet un élargissement de la section, destiné à limiter la vélocité ascensionnelle des fluides. Cette section élargie favorise une décantation rapide des agrégats microbiens. Le milieu de culture est introduit par différents orifices d'alimentation situés au-dessous de l'élargissement de la section. Le liquide produit quitte le fermenteur par un orifice situé au sommet du système, à l'extérieur de la zone de collecte des gaz. On peut prévoir un recyclage des gaz de fermentation, à l'aide d'un compresseur, au moment du démarrage du processus. Le fermenteur est équipé de systèmes adéquats de mesure et de régulation de la température et du pH du milieu de fermentation, ainsi que d'un dispositif de contrôle de mousses.

Pour faciliter l'évacuation de l'anhydride carbonique, on peut envisager d'équiper intérieurement le fermenteur de système de séparation triphasique: gaz, liquide, solide, ou séparateur constitué par une série de cônes tronqués concentriques. Cet emboitement de cônes a deux fonctions, d'une part la face interne du cône canalise le gaz produit, d'autre part cet emboitement crée une zone où la turbulence est très faible, ce qui favorise la décantation des agrégats microbiens sur la face externe du cône. Ces cônes doivent avoir un angle assez important, de façon à faciliter le recyclage des agrégats microbiens.

Au-dessus de l'embouchure du dernier cône, on place un collecteur destiné à récupérer l'ensemble de l'anhydride carbonique produit, qui est évacué au moyen d'une canalisation traversant la paroi du fermenteur.

Ces séparateurs sont agencés verticalement dans le corps du fermenteur, leur nombre étant fonction de sa hauteur.

De plus, la présence de tels séparateurs dans le fermenteur permet d'introduire le concept d'étages théoriques, l'ensemble du système se comportant comme un réacteur multiétagé. On obtient ainsi un modèle théorique du type fermenteur multiétagé dont le premier étage est infiniment mélangé et les autres étages ont un écoulement piston. Cette configuration est optimale pour la plupart des fermentations présentant des phénomènes d'inhibition par le substrat et le produit comme dans le cas de la fermentation alcoolique.

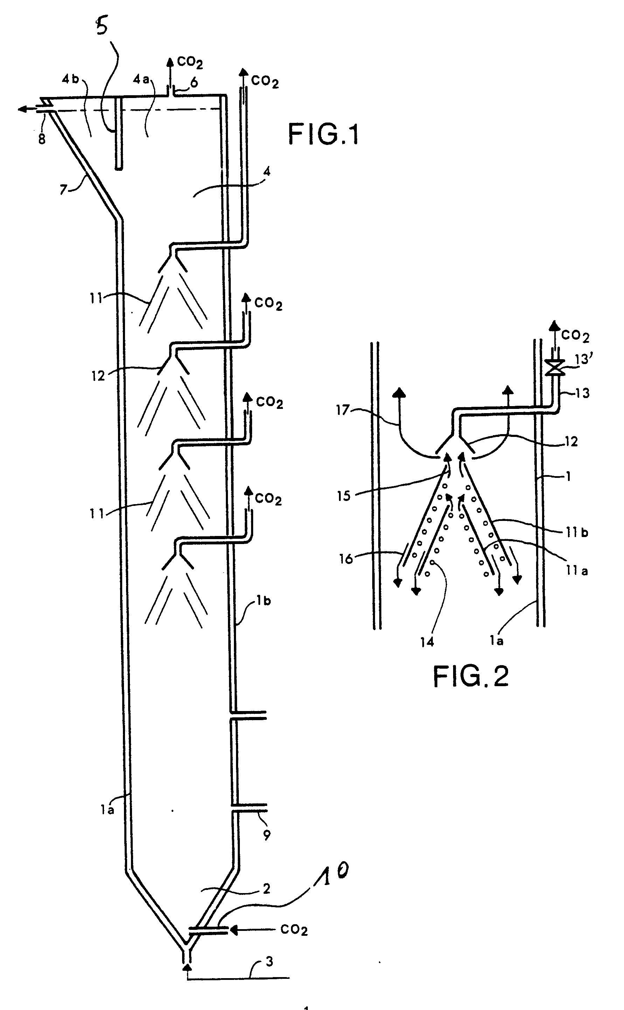

Il est donné à titre d'exemple sur les figures 1 et 2 du dessin annexé une représentation d'un fermenteur vertical tubulaire à double paroi (1a) (1b) avec circulation interparoi de fluide réfrigérant, comprenant le corps cylindrique du fermenteur (1) à circulation verticale des fluides de bas en haut, terminé à la partie inférieure par le cône (2) muni d'une tubulure d'introduction du milieu d'alimentation (3).

Le sommet du fermenteur est constitué par un décanteur (4) à deux sections partiellement séparées par la cloison (5), la première section (4a) dans le prolongement du corps du fermenteur est munie sur le couvercle d'une canalisation d'évacuation de l'anhydride carbonique (6) et la seconde section (4b) évasée vers le haut, adjacente au sommet du fermenteur, dont la paroi inclinée (7) définit avec l'axe du fermenteur (1) un angle d'environ 50 à 60°, est munie d'une canalisation d'évacuation du liquide produit (6). Le corps du fermenteur (1) comporte à divers niveaux plusieurs canalisations d'introduction (9) du milieu d'alimentation et d'une canalisation de recyclage de l'anhydride carbonique (10) mise en circuit au moment du démarrage de la fermentation.

Le corps du fermenteur est garni intérieurement de plusieurs systèmes de séparation triphasiques (11) dits séparateurs et de collecteurs d'anhydride carbonique (12).

Le séparateur (11) comprend une série de cônes tronqués concentriques coaxiaux avec le fermenteur (11a, 11b,...). Les sommets des cônes emboités sont dirigés vers le haut, et les parois parallèles définissent avec l'axe du fermenteur un angle de l'ordre de 50 à 60°C.

La figure 2 met en évidence les fonctions de ce type de séparateur et la circulation des fluides et solides au niveau du séparateur.

L'anhydride carbonique produit, après coalescence des bulles en (14) sur les parois internes des cônes, est canalisé selon les courants gazeux (15), puis collecté par le collecteur (12) surmontant la partie supérieure de la série de cônes. L'anhydride carbonique collecté est évacué par la canalisation d'évacuation (13), raccordée au collecteur (12). Cette canalisation d'évacuation des gaz de fermentation, qui peut être interne ou externe au corps du fermenteur, est munie d'un moyen de contrôle du niveau du liquide. L'évacuation de l'anhydride carbonique peut être contrôlée par pressostat (13'), réglé sur la profondeur à laquelle se fait l'évacuation, ou capteur de niveau. Les agrégats microbiens décantent sur les faces externes des cônes (11a) (11b) et sont recyclés selon les directions (16) vers le bas du fermenteur. Le liquide circule verticalement selon la direction (17).

Une série d'essais a été réalisée dans un fermenteur constitué d'une colonne en verre de 4,6 cm de diamètre intérieur, de hauteur totale 47,5 cm, surmontée d'une partie évasée de 13,3 cm de diamètre intérieur. La cloche de collecte des gaz au centre de la partie évasée a un diamètre intérieur de 4,6 cm. Les capteurs de température et de pH sont disposés à mi-hauteur de la colonne.

Le volume total du système est de 1,2 litre. Le volume, non optimisé, de l'anneau de décantation est de 0,4 litre et le volume utile du fermenteur, renfermant la totalité de la population microbienne, de 0,8 litre.

Deux points d'alimentation en milieu de culture sont prévus à la base et à la mi-hauteur de la colonne.

On maintient constant le niveau du liquide dans le fermenteur par écoulement au travers d'un orifice de surverse disposé dans la partie annulaire de décantation.

Les gaz de fermentation, recueillis au niveau de la cloche de collecte sont évacués par un orifice indépendant du précédent. Une partie des gaz est réinjectée à l'aide d'un compresseur, à la base de la colonne au travers de six injecteurs à raison de 300 ml/mn. L'autre partie est évacuée au travers d'un rotamètre qui donne une indication de la production en anhydride carbonique et donc en éthanol.

On maintient la température à 33°C par circulation d'un liquide caloporteur dans une tubulure enroulée autour de la colonne. On préchauffe le milieu d'alimentation à la même température. En outre, le pH est régulé à 5 par apport d'une solution 2N d'hydroxyde de sodium au niveau de la cloche de collecte des gaz. On contrôle la formation de mousses par apport asservi d'un agent tensio-actif.

On constitue le milieu de culture par une solution synthétique de composition suivante:

| | |
|---|---|
| . glucose | 100 à 120 g/l |
| . sulfate d'ammonium | 1 g/l |
| . phosphate de potassium monopotassique | 1 g/l |
| . sulfate de magnésium | 2,5 g/l |
| . extrait de levure | 2,5 g/l |

On effectue la détermination de la concentration alcoolique par chromatographie en phase gazeuse, et la détermination de la quantité de sucre résiduel par chromatographie liquide haute performance.

**Essai 1**

Le fermenteur rempli de milieu de culture et dans les conditions précitées, est innoculé. Après constitution de la biomasse, il est alimenté à sa base avec un débit de 2 l/h (soit un taux de dilution de 2,5 h⁻¹). Les paramètres d'état relevés en régime permanent sont les suivants:

| | |
|---|---|
| Substrat initial | 100 g/l |
| Substrat dans l'effluent | 0 g/l |
| Ethanol dans l'effluent | 51 g/l |
| Biomasse dans le réacteur | 51 g/l |
| Biomasse dans l'effluent | 1 g/l |

La vitesse de production du gaz carbonique est 850 ml/min, soit 117 g de $CO_2$ par litre de volume utile et par heure. La productivité en éthanol est 127 g par litre de volume utile et par heure. Le taux de croissance des microorganismes est voisin de 0,05 h⁻¹. La productivité specifique est de 2,54 grammes d'éthanol par gramme de biomasse (matière sèche) et par heure. Le rendement de conversion du substrat en éthanol approche le rendement maximal théorique mais la productivité spécifique est faible par rapport aux potentialités des microorganismes (environ 4 grammes éthanol par gramme de biomasse sèche et par heure). Ceci est dû à l'épuisement du milieu en substrat carboné. La durée de ce régime permanent a été volontairement limitée à 50 temps de séjour.

**Essai 2**

Pour lever la limitation due à l'épuisement du substrat carboné, la concentration en glucose du milieu d'alimentation est portée à 116 g/l et le débit d'alimentation à 3,67 l/h (soit un taux de dilution de 4,7 h⁻¹).

Les paramètres d'état relevés en régime permanent sont les suivants:

| | |
|---|---|
| Substrat initial | 116 g/l |
| Substrat dans l'effluent | 15 g/l |
| Ethanol dans l'effluent | 51 g/l |
| Biomasse dans le réacteur | 63 g/l |
| Biomasse dans l'effluent | 0,5 g/l |

La vitesse de production de gaz carbonique est 1700 ml/min, soit 234 g de $CO_2$ par litre de volume utile et par heure. La productivité en éthanol est 240 g par litre de volume utile et par heure.

Le taux de croissance des microorganismes est voisin de 0,04 h⁻¹. La productivité spécifique est de 4 g d'éthanol par g de biomasse (matière sèche) et par heure.

Le rendement de conversion du substrat est proche du rendement maximal théorique.

La durée de ce régime permanent a été volontairement limitée à 100 temps de séjour d'une durée de 20 minutes.

**Essai 3**

Dans cet essai, un second fermenteur est connecté en série avec le premier. De construction géométrique semblable, il présente un volume réactionnel deux fois plus important (2 litres). Ce second fermenteur est alimenté par l'apport continu de milieu fermenté issu du premier fermenteur transportant la fraction de biomasse non retenue (0,5 - 2 g/l) et pour un apport supplémentaire de milieu nutritif concentré, afin d'élever la teneur en glucose. La température est régulée à 30°C; et le pH n'est pas contrôlé.

Pour un débit total de 2,3 l/h et une concentration globale de glucose de 195 g/l, en régime permanent à la sortie du système on a relevé les paramètres d'état suivants:

| | |
|---|---|
| Ethanol dans l'effluent | 81 g/l |
| Substrat dans l'effluent | 29 g/l |
| Biomasse dans l'effluent | 0,7 g/l |

La productivité atteint donc 61 g/l/h. Cette valeur, plus faible que précédemment, résulte de l'inhibition accrue du métabolisme bactérien par la concentration élevée d'éthanol, supérieure à 10° GL; et des conditions opératoires non optimales (débits et rapports des volumes respectifs des étages 1 et 2).

**Essai 4**

Après obtention d'un régime permanent stable, le milieu d'alimentation du fermenteur est substitué par un milieu volontairement contaminé (0,93 g/l de matière sèche) par des germes d'origine aérienne (moisissures, levures, bacilles coques).

On constate une chute de la concentration en éthanol dans l'effluent de 52 à 9 g/l en 80 heures. On observe simultanément un accroissement de la concentration en glucose résiduel jusqu'à 35 g/l. Ceci est principalement le résultat de la perte rapide des flocs de Zymomonas dans l'effluent, causée par une formation de mousse incontrolable.

Après nettoyage et désinfection des circuits d'alimentation, le rétablissement d'une alimentation avec du milieu stérile se traduit par un retour à l'état initial en moins de 100 heures. Des contrôles microscopiques effectués tout au long de l'expérience montrent la disparition progressive des contaminants à l'exception de quelques rares levures restant associées aux agrégats de Zymomonas.

**Revendications**

1. Procédé de production d'anhydride carbonique et d'éthanol par fermentation continue d'un milieu de culture inoculé par des microorganismes du type bactéries appartenant au genre Zymomonas floculante avec rétention et recyclage des microorganismes, la rétention étant réalisée par décantation-recyclage interne obtenue par un ralentissement de la vitesse de circulation des fluides, avec réduction de la turbulence dans la partie supérieure de la zone de fermentation, par un brusque élargissement de la section de la dite zone, caractérisé en ce que le milieu de culture est inoculé par une souche hautement floculante de Zymomonas mobolis, déposée à l'Institut Pasteur sous le numéro I-411, et en ce que les gaz de fermentation sont collectés au centre de la partie supérieure de la zone de fermentation où la turbulence du milieu de fermentation est réduite, avec collecte et évacuation de l'anhydride carbonique produit à différents niveaux de la zone de fermentation.

2. Procédé de production d'anhydride carbonique et d'éthanol selon la revendication 1, caractérisé en ce que l'on procède à une séparation triphasique gaz, liquide, solide en réalisant à différents niveaux de la zone de fermentation, la canalisation des bulles d'anhydride carbonique produit et le recyclage des agrégats microbiens.

3. Procédé de production d'anhydride carbonique et d'éthanol selon la revendication 1 ou 2, caractérisé en ce qu'une fraction des gaz de fermentation est recyclée et réinjectée à la partie inférieure de la zone de fermentation, au moment du démarrage du processus de fermentation.

4. Procédé de production d'anhydride carbonique et d'éthanol par fermentation continue selon une quelconque des revendications 1 à 3, caractérisé en ce que le milieu de culture est maintenu à une température comprise entre 20 et 50°C, de préférence 25 à 40°C, le milieu d'alimentation étant préchauffé à la même température, et le pH du milieu maintenu entre 3,7 et 6,5, de préférence 4,3 à 5,2 par apport d'une solution d'un agent alcalin.

5. Procédé de production d'anhydride carbonique et d'éthanol par fermentation continue selon une quelconque des revendications 1 à 4, caractérisé en ce que le milieu d'alimentation est introduit à divers niveaux de la zone de fermentation, en-dessous de la zone où la turbulence du milieu de fermentation est réduite; le niveau du liquide dans la zone de fermentation étant maintenu constant.

6. Procédé de production d'anhydride carbonique et d'éthanol par fermentation continue selon une quelconque des revendications 1 à 5, caractérisé en ce que la formation de mousses est contrôlée par apport régulé d'un agent tensio-actif.

7. Procédé de production d'anhydride carbonique et d'éthanol par fermentation continue, selon une quelconque des revendications 1 à 6, caractérisé en ce que la fermentation est conduite sous une légère pressurisation inférieure à 3 bars.

8. Procédé de production d'anhydride carbonique et d'éthanol selon une quelconque des revendications 1 à 7, caractérisé en ce que le procédé est mis en oeuvre en systèmes monoétagé, bi ou multiétagé.

9. Appareillage de mise en oeuvre du procédé selon l'une quelconque des revendications 1 à 8, constitué par un fermenteur vertical tubulaire (1) à double paroi (1a) (1b) avec circulation inter-parois de fluide réfrigérant, comprenant le corps cylindrique du fermenteur (1) à circulation verticale des fluides de bas en haut, terminé à la partie inférieure par le cône (2) muni d'une tubulure d'introduction du milieu d'alimentation (3), caractérisé en ce que le sommet du fermenteur est constitué par un décanteur (4) à deux sections partiellement séparées par la cloison (5), la première section (4a) dans le prolongement du corps du fermenteur est munie sur le couvercle d'une canalisation d'évacuation de l'anhydride carbonique (6) et la seconde section (4b) évasée vers le haut, adjacente au sommet du fermenteur, dont la paroi inclinée (7) définit avec l'axe du fermenteur (1) un angle d'environ 50 à 60°, est munie d'une canalisation d'évacuation du liquide produit (8); le corps du fermenteur (1) comporte à divers niveaux plusieurs canalisations d'introduction (9) du milieu d'alimentation, et d'une canalisation de recyclage de l'anhydride carbonique (10), et en outre, le corps du fermenteur (1) est garni intérieurement de plusieurs systèmes de séparation triphasique (11) dits séparateurs et de collecteur d'anhydride carbonique (12).

10. Fermenteur vertical selon la revendication 9, caractérisé en ce que le séparateur (11) comprend une série de cônes tronqués concentriques coaxiaux avec le fermenteur (11a, 11b,...) dont les sommets sont dirigés vers le haut et la partie supérieure de la série de cônes surmontée d'un collecteur d'anhydride carbonique (12) prolongé par une canalisation d'évacuation (13), munie d'un moyen de contrôle du niveau de liquide.

**Patentansprüche**

1. Verfahren zur Herstellung von Kohlendioxid und Ethanol durch kontinuierliche Gärung eines Gärmediums, das mit bakteriellen Mikroorganismen der Art flockende Zymomonas geimpft ist, unter Retention und Rezyklierung der Mikroorganismen, wobei die Retention durch innere Dekantierung-Rezyklierung erfolgt und durch eine Herabsetzung der Umlaufgeschwindigkeit der Fluide bei Verringerung der Turbulenz in dem oberen Teil der Gärzone durch eine plötzliche Vergrößerung des Querschnitts der genannten Zone herbeigeführt wird, dadurch gekennzeichnet, daß das Gärmedium geimpft wird mit einem stark flockenden Stamm von Zymomonas mobolis, hinterlegt beim Institut Pasteur unter der Nummer I-411, und daß die Gärgase in der Mitte des oberen Teils der Gärzone, wo die Turbulenz des Gärmediums herabgesetzt ist, aufgefangen werden, unter Sammeln und Austragen des in den verschiedenen Höhen der Gärzone gebildeten Kohlendioxids.

2. Verfahren zur Herstellung von Kohlendioxid und Ethanol nach Anspruch 1, dadurch gekennzeichnet, daß eine dreiphasge Trennung: Gas, Flüssigkeit, Feststoff vorgenommen wird, indem in verschiedenen Höhen der Gärzone die Ableitung der Blasen von erzeugtem Kohlendioxid und die Rezyklierung der mikrobiellen Aggregationen vorgenommen wird.

3. Verfahren zur Herstellung von Kohlendioxid und Ethanol nach Anspruch 1 oder 2, dadurch gekennzeichnet, daß eine Fraktion der Gärgase zum Zeitpunkt des Beginns des Gärprozesses rezykliert und in den unteren Teil der Gärzone wiedereingeführt wird.

4. Verfahren zur Herstellung von Kohlendioxid und Ethanol durch kontinuierliche Gärung, nach einem der Ansprüche 1 bis 3, dadurch gekennzeichnet, daß das Gärmedium auf einer Temperatur zwischen 20 und 50°C, vorzugsweise zwischen 25 und 40°C, gehalten wird, wobei das Nährmedium auf die gleiche Temperatur erhitzt wird, und der pH des Gärmediums durch Zuführung eines alkalischen Mittels zwischen 3,7 und 6,5, vorzugsweise zwischen 4,3 und 5,2, gehalten wird.

5. Verfahren zur Herstellung von Kohlendioxid und Ethanol durch kontinuierliche Gärung, nach einem der Ansprüche 1 bis 4, dadurch gekennzeichnet, daß das Nährmedium in verschiedenen Niveaus der Gärzone unterhalb der Zone, in der die Turbulenz des Gärmediums verringert ist, eingeführt wird, wobei das Flüssigkeitsniveau in der Gärzone konstant gehalten wird.

6. Verfahren zur Herstellung von Kohlendioxid und Ethanol durch kontinuierliche Gärung, nach einem der Ansprüche 1 bis 5, dadurch gekennzeichnet, daß die Schaumbildung durch Zugabe eines oberflächenaktiven Mittels reguliert wird.

7. Verfahren zur Herstellung von Kohlendioxid und Ethanol durch kontinuierliche Gärung, nach einem der Ansprüche 1 bis 6, dadurch gekennzeichnet, daß die Gärung unter einem geringen Überdruck von weniger als 3 bar vorgenommen wird.

8. Verfahren zur Herstellung von Kohlendioxid und Ethanol nach einem der Ansprüche 1 bis 7, dadurch gekennzeichnet, daß das Verfahren in 1-, 2- oder mehrstufigen Systemen durchgeführt wird.

9. Einrichtung zur Ausübung des Verfahrens nach einem der Ansprüche 1 bis 8, bestehend aus einem vertikalen, rohrförmigen Bioreaktor (1) mit Doppelwänden (1a, 1b), zwischen denen ein Kühlfluid umläuft, umfassend den zylindrischen Hauptteil des Bioreaktors (1) für die Vertikalbewegung von Fluiden von unten nach oben, im unteren Teil begrenzt durch den Kegel (2), der einen Zuführungsstutzen (3) für Nährmedium aufweist, dadurch gekennzeichnet, daß die Spitze des Bioreaktors von einem Dekanteur (4) mit zwei, durch die Trennwand (5) teilweise voneinander getrennten Abschnitten gebildet wird, von denen der erste Abschnitt (4a) in der Verlängerung des Bioreaktor-Hauptteils an seiner Abdeckung eine Abzugsleitung (6) für das Kohlendioxid aufweist und der zweite, an der Spitze des Bioreaktors befindliche, nach oben sich erweiternde Abschnitt (4b), dessen Schrägwand (7) mit der Achse des Bioreaktors (1) einen Winkel zwischen etwa 50 und 60° einschließt, mit einer Abzugsleitung (8) für die erzeugte Flüssigkeit versehen ist, daß der Hauptteil des Bioreaktors (1) in unterschiedlichen Höhen mehrere Zuführungsleitungen (9) für Nährmedium sowie eine Rezyklierungsleitung (10) für Kohlendioxid aufweist, und daß außerdem der Hauptteil des Bioreaktors (1) im Inneren mit mehreren als Separatoren bezeichneten Dreiphasensystemen (11) und mit Kohlendioxid-Kollektoren-Systemen (12) ausgerüstet ist.

10. Vertikal-Bioreaktor nach Anspruch 9, dadurch gekennzeichnet, daß der Separator (11) eine Gruppe von koaxial zu dem Bioreaktor angeordneten konzentrischen Kegelstümpfen (11a, 11b, ) enthält, deren Spitzen nach oben zeigen und über deren oberem Teil ein Kohlendioxid-Kollektor (12) angeordnet ist, der in eine Abzugsleitung (13) übergeht, die mit einer Einrichtung zur Regelung des Flüssigkeitsstandes versehen ist.

**Claims**

1. Process for the production of carbon dioxide and ethanol by continuous fermentation of a culture medium inoculated with micro-organisms of the bacterial type appertaining to the flocculant <u>Zymomonas</u> genus with retention and recycling of the micro-organisms, the retention being effected by internal decanting and recycling obtained by a reduction of the speed of circulation of the fluids, with reduction of the turbulence in the upper part of the fementation zone by a sudden enlargement of the cross-section of the said zone, characterized in that the culture medium is inoculated with a highly flocculant strain of <u>Zymomonas mobilis</u>, deposited at the pasteur Institute under the number I-411, and in that the fermentation gases are collected at the centre of the upper part of the fermenting zone where the turbulence of the fermentation medium is reduced, with collection and withdrawal of the carbon dioxide produced at different levels of the fermentation zone.

2. Process for the production of carbon dioxide and ethanol according to claim 1, characterized in that a three-phase gas -liquid- solid separation is implemented by effecting the ducting of the carbon dioxide bubbles generated and the recycling of the microbial aggregates at different levels of the fermentation zone.

3. Process for the production of carbon dioxide and ethanol according to claim 1 or 2, characterized in that a fraction of the fermentation gases is recycled and reinjected into the lower part of the fermentation zone, at the instant at which the fermentation process is started.

4. Process for the production of carbon dioxide and ethanol by continuous fermentation according to any one of claims 1 to 3, characterized in that the culture medium is kept at a temperature lying between 20 and 50° C, preferably 25 to 40° C, the supply medium being preheated to the same temperature and the pH of the medium maintained between 3.7 and 6.5., and preferably between 4.3 and 5.2, by infeed of a solution of an alkaline agent.

5. Process for the production of carbon dioxide and ethanol by continuous fermentation according to any one of claims 1 to 4, characterized in that the supply medium is fed in at different levels of the fermentation zone, below the zone in which the turbulence of the fermentation medium is reduced, the level of the liquid in the fermentation zone being kept constant.

6. Process for the production of carbon dioxide and ethanol by continuous fermentation according to any one of claims 1 to 5, characterized in that the forming of foams is controlled by controlled infeed of a surface-active agent.

7. Process for the production of carbon dioxide and ethanol by continuous fermentation according to any one of claims 1 to 6 characterized in that the fermentation is conducted under a slight pressurisation of less than 3 bars.

8. Process for the production of carbon dioxide and ethanol according to any one of claims 1 to 7, characterized in that the process is applied in the form of single-stage, two-stage or multistage systems.

9. Apparatus for putting into effect the process according to any one of claims 1 to 8, constituted by vertical tubular fermenter (1) with a double wall (1a) (1b) with circulation of refrigerating fluid between walls, comprising the cylindrical fermenter body (1) for vertical flow of the fluids from the bottom towards the top, delimited in the lower portion by the cone (2) provided with a connector for infeed of the supply medium (3), characterized in that the top of the fermenter is formed by a decanter (4) having two sections partly separated by the partition (5), the first section (4a) in the extention of the fermenter body is provided in the top with a conduit (6) for discharge of the carbon dioxide and the second (4b) diverging towards the top, adjacent to the top of the fermenter, the sloping wall (7) of which defines an angle of approximately 50 to 60° with the axis of the fermenter (1) is provided with a conduit (8) for discharge of the liquid produced, the body of the fermenter (1) has at different levels several pipes (9) for the introduction of the supply medium and a conduit (10) for recycling of the carbon dioxide and furthermore the fermenter body (1) is provided internally with several systems (11) for three-phase separation referred to as separators, and as carbon dioxide collectors (12).

10. Vertical fermenter according to claim 9, characterized in that the separator (11) comprises a series of concentric truncated cones co-axial with the fermenter (11a, 11b...) the apexes of which are upwardly directed, the upper portion of the series of cones being topped by a carbon dioxide collector (12) extended by a discharge pipe (13) provided with means for controlling the level of liquid.

FIG.1

FIG.2